# EUROPEAN PATENT SPECIFICATION

(11) **EP 1 853 325 B1**
(45) Date of publication and mention of the grant of the patent: **27.05.2009**
(21) Application number: 05700602.5
(22) Date of filing: 25.01.2005
(51) Int. Cl.: A61L 2/14, A61L 2/20, A61L 2/24, A61L 2/26, A61L 9/22, A61B 19/00, C01B 13/10

(54) **A METHOD AND AN APPARATUS FOR STERILIZATION OF AN ITEM**
VERFAHREN UND VORRICHTUNG ZUR STERILISIERUNG EINES GEGENSTANDS
PROCEDE ET DISPOSITIF DE STERILISATION D'UN ARTICLE

(43) Date of publication of application: 14.11.2007
(73) Proprietor: Bat Holding ApS, 2000 Frederiksberg C (DK)
(72) Inventor: BUSTED, Tommy, 2000 Frederiksberg C (DK)
(74) Representative: HOEIBERG A/S
(86) International application number: PCT/DK2005/000052
(87) International publication number: WO 2006/079337

(56) References cited:
- EP-A- 1 356 828
- WO-A-99/53966
- WO-A-03/039607
- DE-A1- 3 634 538
- US-A- 5 055 115
- US-A1- 2003 198 571
- US-A1- 2004 022 673
- US-B1- 6 572 817
- DATABASE WPI Section PQ, Week 200507 Derwent Publications Ltd., London, GB; Class P34, AN 2005-062635 XP002323501 & KR 2004 077 089 A (UHM H S) 4 September 2004 (2004-09-04)
- RON NICKERSON ET AL.: "Critical cleaning in precision manufacturing: Plasma cleaning of medical devices" INTERNET ARTICLE, [Online] June 2000 (2000-06), XP002323500 Retrieved from the Internet: URL:http://www.astp.com/PDFs/PS_medical_cl eaning.pdf> [retrieved on 2005-04-05]

## Description

The present invention relates to a method and an apparatus for sterilization of at least one member with ozone-containing gas.

The method and apparatus is in particular suited for sterilization of members such as reusable medical equipment, in particular heat-sensitive equipment such as endoscopes or ultrasound transducers, but are not limited to such heat-sensitive equipment; it may be dental utensils, surgical instruments, hearing aids, or other medical equipment.

Sterilization is the process of destruction of any vira, bacteriae, fungi or other microorganisms, whether in a vegetative or in a dormant spore state. Conventional sterile processing procedures for medical instruments are mainly autoclave processes involving low pressure and high temperature (such as steam units with a temperature around 140°C - 300°C and pressure around 1 atm below, or dry heat units with higher temperatures) or processes involving toxic chemicals (such as ethylene oxide gas, EO).

Steam pressure sterilization has proven effective, is relatively fast (1-2 hours) and cost effective. However, the autoclave process destroys heat sensitive instruments, such as arthroscopes, endoscopes, and ultrasound transducers, which are adversely affected by the heat due to the different materials being used, these materials having different coefficients of thermal expansion, destroying sensitive parts such as lenses, delicate electrical connections and circuits, and ceramic sound emitters. Further, due to the low pressure of the process, air bubbles may be formed in existing thin water and air ducts, if both ends of the capillary ducts are subjected to under pressure simultaneously, which air bubbles increases the risk of bacteria formation.

Ethylene oxide sterilization is used to cold sterilize heat sensitive instruments in a process involving several vacuum cycles and a heat treatment around 80°C in vacuum, a gas treatment for several hours and a long (two week) aeration phase, in which the gas is to dissipate. This complex and time consuming sterilization process is as such expensive, and additionally it is hazardous, as ethylene oxide is carcinogenic, neurotoxic and highly flammable, requiring ventilation, containment facilities, combustion of the excess gas, and fire protection.

Other existing sterilization processes which are not suitable for such heat-sensitive instruments are gamma radiation sterilization, which due to the radiation effects a change in atomic structure especially in plastic materials, i.e. the materials becomes brittle; and plasma sterilization which due to a high electrical field strength, hydrogen peroxide and vacuum destroys electronic equipment and rubber. Further, said processes require complex devices and work protection for the operators.

A more efficient, safer, and less expensive sterilization process involves the use of ozone O₃, which is an extremely oxidising agent, and which may be easily generated from and transformed back into non-hazardous oxygen O₂, which is a constituent of the surrounding air or at a medical facility is readily available from an oxygen source. By the oxidisation reaction, the ozone is transformed into oxygen, leaving no hazardous residue upon the treated equipment. For conventional sterilization by ozone gas a relatively high concentration of ozone gas is required to sterilize microorganisms and those high concentrations of ozone gas have to be combined with critical levels of humidity because the activity of the ozone increases rapidly with increased relative humidity. Further, the resistance of spores to ozone activity varies from strain to strain, but the differences become relatively small at high relative humidity, which implies, that a high humidity is required for the ozone to be able to penetrate the protective "shell" of such microorganisms. One strain, *Bacillus Subtilis var. Niger* (ATCC 9372) is particularly resistant and as such may be used as an indicator of sterilizer effect.

The US patent application US 200410022673 provides a sterilization process including the steps of introducing an oxidizing agent in a gaseous state into a sterilization chamber or area to be sterilized and introducing gas plasma into the sterilization chamber or area to be sterilized. An apparatus for carrying out the process is also disclosed. The electrodes providing connected to the sterilization chamber are shielded from the sterilization load.

International application WO00/66186 discloses a method and an apparatus comprising a chamber being placed under vacuum during the supply of water vapour and ozone-containing gas, the member to be sterilized being kept there for a predetermined time period in a relative humidity above 95%, preferably at the saturation point at ambient temperature, after which the vacuum is released, lowering the boiling temperature of water. The problem here is the relatively high vacuum needed for said process, which induces air bubbles in ducts as mentioned above, and more importantly, requires specialized equipment to accurately control said humidity, temperature and vacuum, and to contain such a vacuum, which increases the cost of the disclosed apparatus.

Accordingly, it is the object of the present invention to provide a method and an apparatus for sterilization of a member with ozone-containing gas, which do not yield the above disadvantages, and which provides for a rapid, safe and inexpensive sterilization of such member.

This object is achieved by a method and an apparatus for sterilization of at least one member with ozone gas according to the independent claims.

Hereby, a substantial increase in sterilization efficiency is achieved because the ionized ozone gas is able to make contact with the members to be sterilized on all available surfaces, even small or complex surfaces, due to the electrical potential difference between a member and the ionized ozone gas. This results in two advantages; firstly a lower ozone gas amount is required for an effective sterilization of one or more members in the sterilization chamber, e.g. an atmospheric air supply suffices for the production of the required amount of ozone gas. Secondly, a substantial reduction in the required sterilization time is achieved, which is important in e.g. a hospital environment, where time may be an essential factor.

Due to the relatively low temperatures and pressures being utilized by the method and apparatus according to the invention, the sterilized equipment will exhibit reduced deformations and wear, and the equipment is ready for use substantially immediately after sterilization. This is especially advantageous when sterilizing complex medical devices such as ultrasound transducers and endoscopes comprising different materials, because the sterilization wear of these devices have been reduced significantly, which results in longer durability of these often very costly devices, which effectively reduces their operational cost. Indeed, even other non-medical types of equipment such as reusable hygienic articles may be sterilized effectively by the present invention, because of the relatively low temperature and pressures involved and the utilization of ozone gas as reactive sterilization agent.

Further, it is a safer and more cost effective sterilization, because it is not necessary to have specific or costly safety measures installed at or around the sterilization apparatus, and because no complex control systems are needed to monitor and precisely control the time, pressure and temperature present in the sterilization chamber.

The invention will be described in detail in the following in relation to one exemplary embodiment of the present invention and with reference to the accompanying schematic drawings, in which:
- Fig.1: is an overview of parts in a sterilization apparatus according to one embodiment of the invention, and
- Fig. 2: is a perspective view seen from above of the apparatus of Fig. 1 included in a cabinet comprising a detachably connected cassette housing.

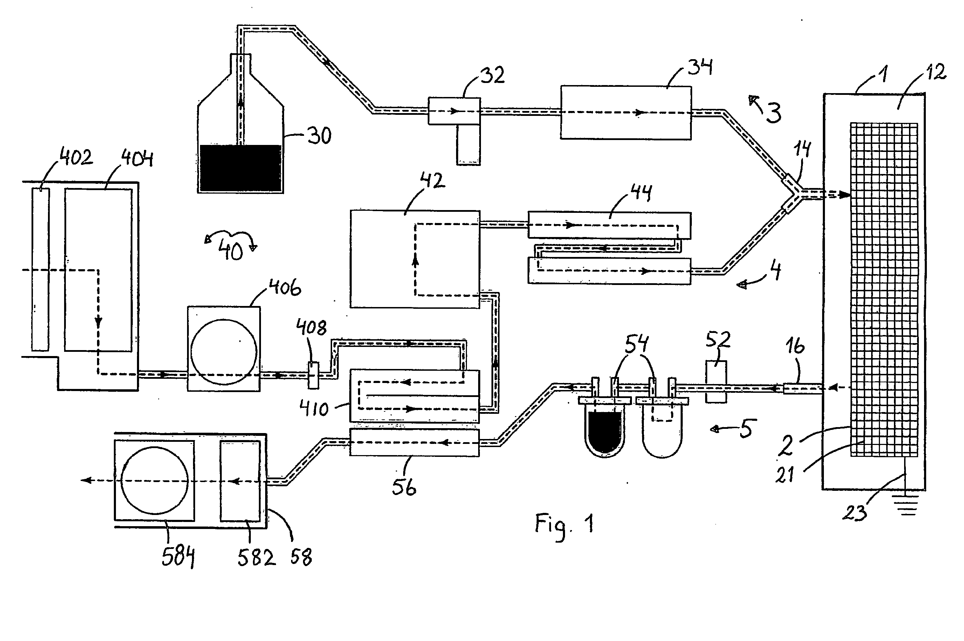

In figure 1 is shown an overview of a sterilization apparatus according to one embodiment of the invention. Generally, the apparatus comprises a housing 1 having a sterilization chamber 12 accommodating a grounded metal grate 2, a water vapour production facility 3, an ionized ozone production facility 4, and an outlet gas cleaning facility 5 for removal of the outlet fumes from the sterilization chamber 12.

The housing 1 comprises a sterilization chamber 12, in which a metal grate 21 during use of the apparatus provides a first electrical potential, being a ground potential 23, to medical equipment (not shown), which is placed upon said grate to be sterilized. Said housing 1 is capable of being opened and closed in order to place and remove said medical equipment before and after sterilization.

The water vapour production facility 3 comprises a supply 30 of demineralised water, which is connected to a water dosage pump 32 and a steam generator 34, both being of a type known to the skilled person, for the production of water steam to an intake valve 14 leading into the sterilization chamber 12. The precise temperature of the water vapour is not important to the effectiveness of the sterilization apparatus or method according to the invention, but the humidity level may be around 80-100%. As an alternative to water vapour, any other electrically conductive vapour may be utilized.

The ionized ozone production facility 4 comprises a clean air intake assembly 40 comprising an air filter assembly 402, 404 connected to an air intake pump 406, a Teflon filter 408 and a UV/C radiation unit 410. For supplying a clean gas without contaminants the surrounding air is taken in through a HEPA filter 402 and an active coal filter 404. The air intake pump 406 may be any conventional type providing a suitable amount of air for the production of a continuous gas flow. The Teflon filter 408 removes any particulates in the air flow. The UV/C radiation unit 410 radiates in a conventional way the air going through in order to destroy any remaining bacteriae or microorganisms contained in the passing air with ultra violet radiation.

The facility 4 further comprises an air ionization or charging unit 42 and an ozone generator 44, which based on filtered ionized intake air produces ionized ozone-containing gas for supply via the opening of said intake valve 14 into said sterilization chamber 12.

In the air ionization unit 42 the incoming clean air is ionized or charged by a corona current, i.e. a high electrostatic charge is provided to the incoming air by a high voltage electrode being placed in the air flow. Electrons are exited by the electrode and transferred through the air, whereupon the passing air is laden with electrons and thus is being charged or ionized, the second electrical potential being attained in the order of 80 kV (40 µA). Even if ozone as a side effect is created in the process, it is not the main objective of this treatment.

Then, the ionized air is supplied to the ozone generator 44, which in a conventional way generates ozone gas by subjecting said ionized air to a short wave length UV radiation or UV/C radiation around 185 nm. Alternatively, any other type of conventional ozone production unit may be utilized. The UV/C radiation induces a photochemical reaction of the oxygen, which is present in the intake air, thus producing a continuous flow of air containing ozone or O₃ gas, i.e. a highly reactive gas of molecules consisting of three oxygen atoms in the form of a triangle, each angle being 117 degrees. Since the oxygen containing gas, prior to ozone production, was ionized to a relatively high electrical potential, the resulting ozone or O₃ gas in the air is charged with electrons, creating O₃⁻.

Thus, ionized ozone gas O₃⁻ is produced effectively in a filtered, sterilized, ionized air supply. Such ionized ozone gas O₃⁻ is very aggressive as compared to non-ionized ozone gas, which conventional ozone sterilization apparatus are using presently. The aggressive O₃⁻ effectively destroys the bacteria upon the medical equipment, and reduces the need for a high concentration of ozone during sterilization.

The inlet valve 14 is preferably supplying both water vapour and ionized ozone gas containing air to the sterilization chamber 12 for a simplification of the construction and for facilitating the detachable connection of the housing, as will be described below. Alternatively, a combined inlet/outlet valve or two or more separate inlet and/or outlet valves 14, 16 may be provided.

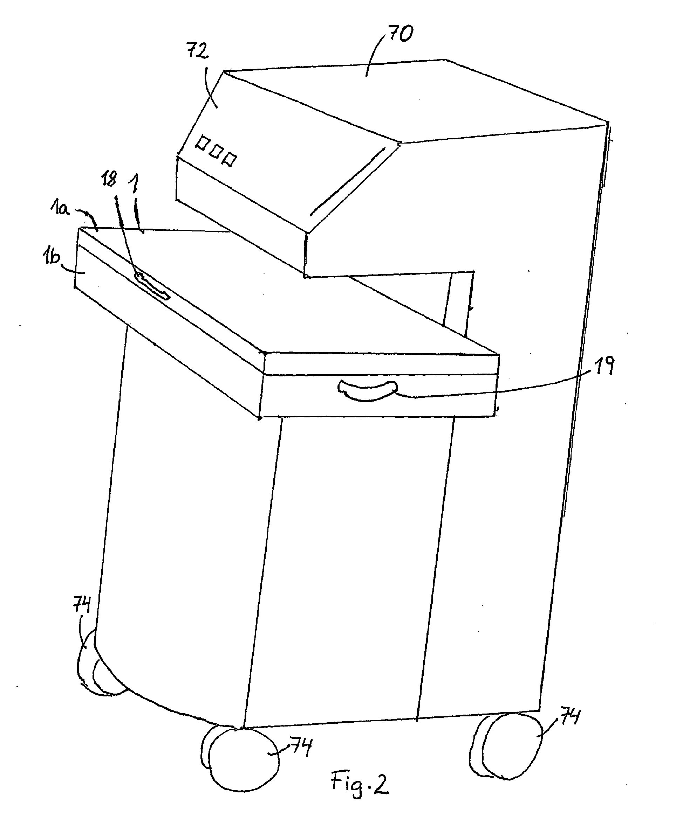

In the embodiment shown in fig. 1 and 2, the water vapour is fed through the inlet valve 14 and after a predetermined vapour exposure time period, e.g. in the order of 1 minute, expelled through the outlet valve 16. Due to the fact that the water vapour effectively covers accessible surfaces of medical equipment present on the metal grate 21, the first electrical potential created by the metal grate 21 is transferred by the electrically conductive water vapour layer to substantially all of said surfaces, even non-conducting surfaces like rubber ones.

After expelling the water vapour, air containing ionized ozone gas is supplied to the sterilization chamber via the inlet valve 14. The ionized ozone gas is effectively and substantially instantly attracted to the grounded equipment, resulting in an extremely effective equipment surface covering of said ionized ozone gas, which results in the highly effective sterilization effect provided by the method and apparatus according to the invention.

Further, because the ozone has been ionized to a second electrical potential prior to introduction to the sterilization chamber 12, the ionized ozone gas molecules O₃⁻ will substantially only be attracted to the charged surfaces, i.e. the medical equipment surfaces and the metal grate 21, and not to the inner sides of the housing 1. This further improves the efficiency of the ozone sterilization, which also provides for a further reduced ozone gas amount need in order to achieve an effective sterilization. This also provides an added advantage, because the major part of the ionized ozone gas will be catalytically converted back into oxygen because of contact with said surfaces, reducing the need for containment and/or conversion of the exhaust air from the sterilization chamber 1 in order to reduce the amount of remaining aggressive ozone gas in the exhaust air.

In order to reduce the ionized ozone gas amount, which is being attracted to the grounded grate, the surface area of the grounded part of the grate may preferably be kept to a minimum, e.g. by manufacturing the grate from a thin wire, having large interspaces or the like, or providing the grate with isolation on the parts not subjected to the members being sterilized., e.g. depending on type of equipment to be sterilized.

An electrical potential difference is to exist between the equipment surfaces, having the first electrical potential, and the ionized ozone gas, having the second electrical potential, but for an effective sterilization effect, the magnitude of the difference is not important, nor the sign of the potential between the two. Other possible embodiments may comprise a positive potential on the surfaces, or even positively ionized ozone and a negative or ground potential on the surfaces. The metal grate 21 may be made of any type of conductive material, such as surgical stainless steel, other types of stainless steel, iron or copper, and may be of any form and type, e.g. a plate grate, a tray with edges, a basket or the like. Alternatively, the means for providing the electrical potential to the members may also be one or more metal plate, wires, and/or plugs specially adapted to certain medical equipment.

After the ionized ozone gas has worked for a predetermined exposure time period, e.g. in the order of one minute, the gas is expelled, whereupon the cycle of water vapour, and ionized ozone gas is repeated a predetermined number of times, e.g. 6 times, and then a water vapour exposure is performed as an final step, also in order to catalytically change the remaining ionized ozone back into oxygen.

The outlet gas cleaning facility 5 comprises an outlet valve 16, a flow sensor 52, moisture separators 54, a UV/C radiation unit 56, and an exit unit comprising a carbon filter 582 and an air outlet pump 584. During and/or after sterilization, the gas or air inside the sterilization chamber 12 is expelled through the outlet valve 16, either by opening this actively or by keeping it opened continually. The flow sensor 52 registers the air flow amount. The moisture separators 54 removes humidity from the expelled air, and reduces the risk of airborne contaminants therein. In order to ensure all ozone has been catalytically converted into oxygen and no live microorganisms are present in the exhaust gas, the outlet gas flow is transferred to the UV/C radiation unit 56. The outlet gas is then removed from the apparatus by the exit unit 58, where an air pump 584 withdraws the gas through the carbon filter 58, which removes any remaining contaminants in the expelled air, and even inhibits outside contaminants to enter the apparatus during shut down.

Preferably, said water vapour is supplied for a predetermined vaporization time period and then expelled; and said ionized ozone gas supplied and expelled after said predetermined exposure time period; and this is repeated a predetermined number of times. As an alternative to the alternating water vapour and ionized gas cycle, the ionized ozone gas may be made to flow through the sterilization chamber after the first water vapour exposure, either continuously or intermittently. Further, the ionized ozone gas and the water vapour may be supplied at the same time, even though this seriously affects the efficiency of the ionized ozone gas, because the ozone gas may thus be bound to the water vapour in the space inside the sterilization chamber and not to the equipment.

Each total sterilization time period, i.e. total number of cycles and final water vapour step, may last from 1 to 120 minutes, preferably from 10 to 20 minutes, and more preferably 13 to 15 minutes. Example of such a time period is one minute at a time, alternating between water vapour and ionized ozone gas, optionally having a small hold period or suction period in the interval of tenths of seconds to tens of seconds between gas flows, in total 7 times water vapour exposure and 6 times ionized ozone exposure. The hold period provides for a rest period for the water vapour to settle on the equipment to be sterilized, whereupon or during which hold period the remaining water vapour in the sterilization chamber is sucked out thereof.

A problem applicant has not seen described in prior art is how to contain, i.e. hold and/or transport the sterilized members and keeping these in such a sterilized state, e.g. during storage, until their required use. Accordingly, a housing is provided, which is suitable for use with an apparatus according to the present invention or for performing the method according to the present invention, and which may contain the members in a sterilized state until use of said members is required.

Advantageously, the apparatus is provided with means for providing a pressure difference between said sterilization chamber and the surroundings, such that the pressure is higher inside the sterilization chamber than in the air surrounding the housing. This may be provided by designing the inlet and outlet valves 14, 16 in such a way that a slight over pressure is present in the sterilization chamber 12 during and/or especially after sterilization, in order to keep a sterile environment by preventing contaminants from entering via the surrounding air.

Preferably, said inlet valve 14 and outlet valve 16 may be individually controllable in order to provide said pressure difference, and/or said water dosage pump 32, said air intake pump 406 and said air outlet pump 584 may be individually controllable.

Preferably, said controlling is performed by control means such as a processor or the like, also comprised in the apparatus.

In Fig. 2 is shown a perspective view from above of a compact and self-contained apparatus according to the present invention, in which all the constituting parts shown in Fig. 1 may preferably be included in a cabinet 70, which is provided with wheels 74 for easy transport of said apparatus. Thus, the only external supply needed is an electrical power unit, e.g. mains, in which case the apparatus further is provided with a high voltage generator for supply of e.g. the UV radiation units and the ionization unit. The water may also be supplied from an external demineralised water supply, but is preferably supplied from a water supply from inside the cabinet 70.

The front side of said cabinet 70 is provided with a control panel 72, from which an operator is able to control start and end of the sterilization process, or set parameters such as selection of preset sterilization programs, control and monitor temperature, pressure, flow of exhaust gas, ozone gas amount, and/or other functions to control and preferably electronically record the sterilization process within the sterilization chamber inside the housing 1.

Alternatively, in a simplified embodiment, no panel is provided because the attachment of the cassette housing 1 to the cabinet 70 automatically starts a preset sterilization programme, which ends after a predetermined time period, and hereafter a small over pressure is supplied to the sterilization chamber, which then may be opened by the use of an opening handle 18, which is provided on the top part of the cassette housing. Surveillance parameters from the sterilization process may be provided visually, e.g. on a screen or with indicator lights.

The housing 1 is a cassette housing having a sterilization chamber for receiving at least one member, said sterilization chamber comprising means for providing said at least one member with a first electrical potential, said cassette housing comprising an inlet valve 14 for supplying water vapour and for supplying ionized ozone gas and an outlet valve 16 for exhaust gasses. Further, said cassette housing may be provided with means for providing a pressure difference between said sterilization chamber and the surroundings, such that the pressure is higher inside the sterilization chamber, as described above.

In fig. 2, the housing 1 is provided as a cassette housing or "suit case" having a top part 1a and a bottom part 1b mutually attached by hinges (not shown) on one side thereof. Said cassette housing is detachably connected to the cabinet 70 via attachment means comprising the inlet and outlet valves 14, 16, which enable said housing to be detachably connected to said apparatus. The cassette housing is provided with transport handles 19 on each side of it for transporting the cassette housing per se away from the apparatus and keeping the members or medical equipment onto the horizontally placed metal grate inside, either for storage or for being utilized directly on site or elsewhere in the medical facility.

According to the "Premarket Notifications [510(k)] for Biological Indicators Intended to Monitor Sterilizers used in Health Care facilities; Draft Guidance for Industry and FDA Reviewers", published by the US Federal Drug Administration in 2001, the strain of *Bacillus Subtilis var*. *Niger* may be used as a biological indicator for users to be provided with information on the effectiveness of the sterilizer process. In tests with the apparatus performing a method according to the invention on an apparatus as shown in Fig. 1 and 2, upon medical equipment provided with said bacteria strain the sterilization effectiveness proved very high, at least comparable to the most effective EO and vacuum sterilizers available on the market today.

The housing may be provided in any other way, e.g. a cylindrical bucket with a lock, a closeable plastic bag, or the like; the cabinet may be present or not; the housing may be detachably connected or not; the oxygen may alternatively be provided by an oxygen supply container.

## Claims

1. A method for sterilization of at least one member with ozone gas, including the steps of:
- providing a housing (1) having a sterilization chamber (12) in which at least one member is placed;
- providing said at least one member with a first electrical potential, which is a ground or positive or negative potential,
- supplying water vapour to said sterilization chamber (12);
- electrostatically ionizing an oxygen containing gas to a second electrical potential, which is a positive or negative potential and different from said first electrical potential, in a ionization unit (42);
- based on said electrostatically ionized oxygen containing gas generating ionized ozone gas in a ozone generator (44);
- supplying said ionized ozone gas to said sterilization chamber (12) for attracting said ionized ozone gas to the surfaces of said at least one charged member with said first electrical potential; and
- exposing said at least one member to the ionized ozone gas for a predetermined exposure time period.

2. A method according to claim 1, where water vapour is supplied for a predetermined vaporization time period and then expelled; and said ionized ozone gas supplied and expelled after said predetermined exposure time period; and this is repeated a predetermined number of times.

3. A method according to claim 2, further including a rest period after said predetermined vaporization time period, said rest period lasting in the interval of tenths of a second to tens of seconds.

4. A method according to claim 2 or 3, further ending the sterilization with a water vapour supply for said predetermined vaporization time period.

5. A method according to any of the claims claim 2 to 4, where said predetermined vaporization time period and said predetermined exposure time period lie in the order of 30 sec to 15 minutes, preferably 1 minute to 5 minutes, and most preferably around 1 minute, respectively.

6. A method according to any of the preceding claims, where said first electrical potential is a ground potential (23) and said second electrical potential is a larger negative potential.

7. A method to any of the preceding claims, in that the provision of the first electrical potential is achieved with a metal grate (2) onto which said at least one member is placed.

8. A method according to any of the preceding claims, further comprising providing a pressure difference between said sterilization chamber (12) and the surroundings, such that the pressure is higher inside the sterilization chamber (12).

9. A method according to any of the preceding claims, where said at least one member is exposed to the ionized ozone gas in a continuous gas flow.

10. A method according to any of the claims 1-8, where said at least one member is exposed to the ionized ozone gas in an intermittent gas flow.

11. A method according to any of the claims 1-9, where said at least one member is exposed to the ionized ozone gas in a one time exposure.

12. A method according to any of the preceding claims, where said at least one member is a complex medical device such as an ultrasound transducer.

13. An apparatus for carrying out the process according to claims 1-12 sterilization of at least one member with ozone gas, comprising
- a housing (1) having an sterilization chamber (12) for receiving said at least one member;
- means for supplying water vapour to said sterilization chamber;
**characterized in, that** said apparatus further comprises
- means for providing said at least one member with a first electrical potential, which is a ground or positive or negative potential
- means for supplying an electrostatically ionized ozone gas to said sterilization chamber (12), comprising
- an oxygen containing gas supply connected to
- an electrostatic gas ionization unit (42) for providing said gas with a second electrical potential, which is a positive or negative potential, wherein the first potential is different from the second one, the gas ionization unit (42) being connected to
- an ozone generator (44) connected to said sterilization chamber (12).

14. An apparatus according to claim 13, where said means for providing said at least one member with a first electrical potential is a metal grate (2).

15. An apparatus according to any of the claims 13 and 14, said housing (1) further comprising an inlet valve (14) for said ionized ozone gas and said water vapour, and an outlet valve (16) for the exhaust gasses.

16. An apparatus according to any of the claims 13 to 15, said oxygen containing gas supply being atmospheric air surrounding the apparatus.

17. An apparatus according to any of the claims 13-16, comprising attachment means, which enable said housing (1) to be detachably connected to said apparatus.

18. An apparatus according to claim 17, where said attachment means comprises the inlet (14) and outlet (16) valves.

19. An apparatus according to any of the claims 13 and 14, where said at least one member is a complex medical device such as an ultrasound transducer.

## Patentansprüche

1. Verfahren zur Sterilisation von zumindest einem Element mit Ozongas, umfassend die Schritte:
Bereitstellen eines Gehäuses (1) mit einer Sterilisationskammer (12), in die zumindest ein Element platziert wird;
Bereitstellen des zumindest einen Elements mit einem ersten elektrischen Potential, das ein Erd- oder positives oder negatives Potential ist,
Zuführen von Wasserdampf zu der Sterilisationskammer (12);
Elektrostatisches Ionisieren eines sauerstoffhaltigen Gases zu einem zweiten elektrischen Potential, das ein positives oder negatives Potential ist und von dem ersten elektrischen Potential differiert, in einer Ionisationseinheit (42);
basierend auf dem elektrostatisch ionisierten sauerstoffhaltigen Gaserzeugen von ionisiertem Ozongas in einem Ozongenerator (44);
Zuführen des ionisierten Ozongases zu der Sterilisationskammer (12) zum Anziehen des ionisierten Ozongases zu den Oberflächen des zumindest einen geladenen Elements mit dem ersten elektrischen Potential; und
Aussetzen des zumindest einen Elements bezüglich dem ionisierten Ozongas für eine vorbestimmte Aussetzzeitspanne.

2. Verfahren gemäß Anspruch 1, wobei Wasserdampf zugeführt wird für eine vorbestimmte Verdampfungszeitspanne und dann ausgestoßen wird; und das ionisierte Ozongas zugeführt und ausgestoßen wird nach der vorbestimmten Aussetzzeitspanne; und dies eine vorbestimmte Anzahl wiederholt wird.

3. Verfahren gemäß Anspruch 2, ferner umfassend eine Ruhezeit nach der vorbestimmten Verdampfungszeitspanne, wobei die Ruhezeit sich im Intervall von Zehntelsekunden bis einigen zehn Sekunden bewegt.

4. Verfahren gemäß Anspruch 2 oder 3, ferner umfassend ein Beenden der Sterilisation mit einer Wasserdampfzufuhr für die vorbestimmte Verdampfungszeitspanne.

5. Verfahren gemäß einem der Ansprüche 2 bis 4, wobei die vorbestimmte Verdampfungszeitspanne und die vorbestimmte Aussetzzeitspanne jeweils in der Größenordnung von 30 Sekunden bis 15 Minuten liegt, vorzugsweise 1 Minute bis 5 Minuten und am Bevorzugtesten ungefähr 1 Minute.

6. Verfahren gemäß einem der vorherigen Ansprüche, wobei das erste elektrische Potential ein Erdpotential (23) ist und das zweite elektrische Potential ein größeres negatives Potential ist.

7. Verfahren gemäß einem der vorherigen Ansprüche, wobei das Vorsehen des ersten elektrischen Potentials erzielt wird mit einem Metallgitter (2), auf das das zumindest eine Element platziert wird.

8. Verfahren gemäß einem der vorherigen Ansprüche, ferner umfassend ein Bereitstellen einer Druckdifferenz zwischen der Sterilisationskammer (12) und der Umgebung, so dass der Druck in der Sterilisationskammer (12) höher ist.

9. Verfahren gemäß einem der vorherigen Ansprüche, wobei das zumindest eine Element dem ionisierten Ozongas in einem kontinuierlichen Gasfluss ausgesetzt wird.

10. Verfahren gemäß einem der Ansprüche 1 bis 8, wobei das zumindest eine Element dem ionisierten Ozongas in einem intermittierenden Gasfluss ausgesetzt wird.

11. Verfahren gemäß einem der Ansprüche 1 bis 9, wobei das zumindest eine Element dem ionisierten Ozongas in einer einmaligen Aussetzung ausgesetzt wird.

12. Verfahren gemäß einem der vorherigen Ansprüche, wobei das zumindest eine Element eine komplexe medizinische Vorrichtung ist, wie beispielsweise ein Ultraschallwandler.

13. Vorrichtung zum Ausführen des Prozesses gemäß einem der Ansprüche 1 bis 12 zur Sterilisation von zumindest einem Element mit Ozongas, umfassend ein Gehäuse (1) mit einer Sterilisationskammer (12) zum Aufnehmen des zumindest einen Elements;
Mittel zum Zuführen von Wasserdampf zu der Sterilisationskammer;
**dadurch gekennzeichnet, dass** die Vorrichtung ferner folgendes umfasst:
Mittel zum Bereitstellen des zumindest einen Elements mit einem ersten elektrischen Potential, das ein Erd- oder positives oder negatives Potential ist,
Mittel zum Zuführen eines elektrostatisch ionisierten Ozongases zu der Sterilisations kammer (12), umfassend
- eine sauerstoffhaltige Gaszufuhr, verbunden mit
- einer elektrostatischen Gasionisationseinheit (42) zum Bereitstellen des Gases mit einem zweiten elektrischen Potential, das ein positives oder negatives Potential ist, wobei das erste Potential von dem zweiten differiert, wobei die Gasionisationseinheit (42) verbunden ist mit
- einem Ozongenerator (44), verbunden mit der Sterilisationskammer (12).

14. Vorrichtung gemäß Anspruch 13, wobei das Mittel zum Bereitstellen des zumindest einen Elements mit einem ersten elektrischen Potential ein Metallgitter (2) ist.

15. Vorrichtung gemäß einem der Ansprüche 13 und 14, wobei das Gehäuse (1) ferner ein Einlassventil (14) umfasst für das ionisierte Ozongas und den Wasserdampf und ein Auslassventil (16) für Abgase.

16. Vorrichtung gemäß einem der Ansprüche 13 bis 15, wobei die sauerstoffhaltige Gaszufuhr Außenluft ist, die die Vorrichtung umgibt.

17. Vorrichtung gemäß einem der Ansprüche 13 bis 16, umfassend Anbringmittel, die dem Gehäuse (1) ermöglichen, abnehmbar mit der Vorrichtung verbunden zu werden.

18. Vorrichtung gemäß Anspruch 17, wobei die Anbringmittel das Einlassventil (14) und das Auslassventil (16) umfassen.

19. Vorrichtung gemäß einem der Ansprüche 13 und 14, wobei das zumindest eine Element eine komplexe medizinische Vorrichtung ist, wie beispielsweise ein Ultraschallwandler.

## Revendications

1. Procédé de stérilisation d'au moins un article avec du gaz d'ozone, comprenant les étapes consistant à :
- fournir un logement (1) ayant une chambre de stérilisation (12) dans laquelle au moins un article est placé ;
- fournir au dit au moins un article un premier potentiel électrique qui est un potentiel de masse ou positif ou négatif ;
- fournir de la vapeur d'eau dans ladite chambre de stérilisation (12) ;
- **ioniser de manière électrostatique un gaz** contenant de l'oxygène à un deuxième potentiel électrique, qui est un potentiel positif ou négatif et différent dudit premier potentiel électrique, dans une unité d'ionisation (42) ;
- sur la base dudit gaz contenant de l'oxygène ionisé de manière électrostatique, générer du gaz d'ozone ionisé dans un générateur d'ozone (44) ;
- fournir ledit gaz d'ozone ionisé dans ladite chambre de stérilisation (12) pour attirer ledit gaz d'ozone ionisé vers les surfaces dudit au moins un article chargé au dit premier potentiel électrique ; et
- exposer ledit au moins un article au gaz d'ozone ionisé pendant une période de temps d'exposition prédéterminée.

2. Procédé selon la revendication 1, dans lequel de la vapeur d'eau est fournie pendant une période de temps de vaporisation prédéterminée puis est expulsée ; et ledit gaz d'ozone ionisé fourni est expulsé après ladite période de temps d'exposition prédéterminée ; et cela est répété un nombre de fois prédéterminé.

3. Procédé selon la revendication 2, comprenant en outre une période de repos après ladite période de temps de vaporisation prédéterminée, ladite période de repos durant dans l'intervalle de quelques dixièmes de seconde à quelques dizaines de secondes.

4. Procédé selon la revendication 2 ou 3, comprenant en outre l'étape consistant à terminer la stérilisation avec une alimentation de vapeur d'eau pendant ladite période de temps de vaporisation prédéterminée.

5. Procédé selon l'une quelconque des revendications 2 à 4, dans lequel ladite période de temps de vaporisation prédéterminée et ladite période de temps d'exposition prédéterminée sont respectivement de l'ordre de 30 secondes à 15 minutes, de préférence d'1 minute à 5 minutes, et avec encore plus de préférence d'environ 1 minute.

6. Procédé selon l'une quelconque des revendications précédentes, dans lequel ledit premier potentiel électrique est un potentiel de masse (23) et ledit deuxième potentiel électrique est un potentiel négatif supérieur.

7. Procédé selon l'une quelconque des revendications précédentes, **caractérisé en ce que** la fourniture du premier potentiel électrique est réalisée avec une grille métallique (2) sur laquelle est placé ledit au moins un article.

8. Procédé selon l'une quelconque des revendications précédentes, comprenant en outre l'étape consistant à fournir une différence de pression entre ladite chambre de stérilisation (12) et le voisinage, de sorte que la pression soit supérieure à l'intérieur de la chambre de stérilisation (12).

9. Procédé selon l'une quelconque des revendications précédentes, dans lequel ledit au moins un article est exposé au gaz d'ozone ionisé dans un flux de gaz continu.

10. **Procédé selon l'une quelconque des** revendications 1 à 8, dans lequel ledit au moins un article est exposé au gaz d'ozone ionisé dans un flux de gaz intermittent.

11. Procédé selon l'une quelconque des revendications 1 à 9, dans lequel ledit au moins un article est exposé au gaz d'ozone ionisé au cours d'une exposition en une seule fois.

12. Procédé selon l'une quelconque des revendications précédentes, dans lequel ledit au moins un article est un dispositif médical complexe comme un transducteur d'ultrasons.

13. Appareil pour effectuer le procédé selon les revendications 1 à 12 destiné à la stérilisation d'au moins un article avec du gaz d'ozone, comprenant :
- un logement (1) ayant une chambre de stérilisation (12) pour recevoir ledit au moins un article ;
- des moyens pour fournir de la vapeur d'eau dans ladite chambre de stérilisation ;
**caractérisé en ce que** ledit appareil comprend en outre :
- des moyens pour fournir au dit au moins un article un premier potentiel électrique qui est un potentiel de masse ou positif ou négatif ;
- des moyens pour fournir un gaz d'ozone ionisé de manière électrostatique dans ladite chambre de stérilisation (12), comprenant :
- une alimentation de gaz contenant de l'oxygène raccordée à
- une unité d'ionisation de gaz électrostatique (42) pour fournir au dit gaz un deuxième potentiel électrique qui est un potentiel positif ou négatif, dans lequel le premier potentiel est différent du deuxième potentiel, l'unité d'ionisation de gaz (42) étant raccordée à
- un générateur d'ozone (44) raccordé à ladite chambre de stérilisation (12).

14. Appareil selon la revendication 13, dans lequel lesdits moyens pour fournir au dit au moins un article un premier potentiel électrique est une grille métallique (2).

15. **Appareil selon l'une quelconque des** revendications 13 et 14, ledit logement (1) comprenant en outre une vanne d'entrée (14) pour ledit gaz d'ozone ionisé et ladite vapeur d'eau, et une vanne de sortie (16) pour les gaz d'échappement.

16. Appareil selon l'une quelconque des revendications 13 à 15, ladite alimentation de gaz contenant de l'oxygène étant de l'air atmosphérique entourant l'appareil.

17. Appareil selon l'une quelconque des revendications 1 3 à 16, comprenant des moyens d'attachement qui permettent de raccorder de manière détachable ledit logement (1) au dit appareil.

18. Appareil selon la revendication 17, dans lequel lesdits moyens d'attachement comprennent la vanne d'entrée (14) et la vanne de sortie (16).

19. **Appareil selon l'une quelconque des** revendications 13 et 14, dans lequel ledit au moins un article est un dispositif médical complexe comme un transducteur d'ultrasons.
